# EUROPÄISCHE PATENTSCHRIFT

(11) **EP 1 442 258 B1**
(45) Veröffentlichungstag und Bekanntmachung des Hinweises auf die Patenterteilung: **10.12.2008**
(21) Anmeldenummer: 02783428.2
(22) Anmeldetag: 24.10.2002
(51) Int. Cl.: F24F 11/00, F24F 3/16, A61L 9/22

(54) **SICHERHEITSEINRICHTUNG FÜR DIE LUFT IN WENIGSTENS EINEM RAUM EINES GEBÄUDES**
SICHERHEITSEINRICHTUNG FUR DIE LUFT IN WENIGSTENS EINEM RAUM EINES GEBAUDES
DISPOSITIF DE SECURITE DESTINE A L'AIR D'AU MOINS UN LOCAL D'UN BATIMENT

(30) Priorität: 26.10.2001 DE 10153575
(43) Veröffentlichungstag der Anmeldung: 04.08.2004
(73) Patentinhaber: LK Luftqualität AG, 6014 Littau (CH)
(72) Erfinder: FLEISCHER, Werner, CH-6103 Schwarzenberg (CH)
(74) Vertreter: Uhlemann, Henry
(86) Internationale Anmeldenummer: PCT/IB2002/004952
(87) Internationale Veröffentlichungsnummer: WO 2003/036181

(56) Entgegenhaltungen:
- EP-A- 1 125 588
- DE-A- 3 519 145
- DE-A- 19 651 403
- DE-A- 19 700 964
- US-A- 4 726 824
- US-A- 5 292 280
- US-A- 6 161 764
- PATENT ABSTRACTS OF JAPAN vol. 2000, no. 15, 6. April 2001 (2001-04-06) & JP 2000 337681 A (LK LUFTQUALITAET AG), 8. Dezember 2000 (2000-12-08)

## Beschreibung

Die Erfindung betrifft Sicherheitseinrichtungen für die Luft in wenigstens einem Raum eines Gebäudes, wobei der Raum über mindestens einen Anströmkanal zum Ansaugen von Außenluft und über wenigstens eine Vorrichtung zur Luftbehandlung verfügt.

Bekannte Vorrichtungen zur Luftbehandlung in Form von z. B. Klimaanlagen für Gebäude besitzen den Nachteil, dass diese den Menschen vor auftretenden Luftverunreinigungen insbesondere schädlichen Stoffen oder Organismen nicht schützen.

Durch die Druckschrift EP 1 125 588 A2 (Verfahren und Vorrichtung zur Luftbehandlung wenigstens eines Raumes durch Luftionisation) ist ein Verfahren und eine Vorrichtung zur Luftbehandlung wenigstens eines Raumes durch Luftionisation bekannt wobei die Ionisation durch elektrische Entladung in Ionisationsröhren oder in Koronaentladungsröhren erfolgt. Die Vorrichtung besteht dabei im Wesentlichen aus einem Luftströmungsfühler, einem Luftfeuchtefühler, zwei Luftqualitätssensoren und einem Ozonsensor, die mit einem Steuergerät zusammengeschaltet sind. Neben der Luftionisation wird eine Ozonreduzierung über den Ionisationsapparat erreicht. Eine Abschottung des Raumes ist nicht vorgesehen.
Die Druckschrift DE 196 51 403 A1 (Mehrstufiges Luftgütesystem für Fahrzeugkabinen) beschreibt eine Apparatur zur physikalischen Luftaufbereitung mittels Luftionisatoren flacher Bauart zur Aufbereitung der Luft in Fahrzeugkabinen von Automobilen oder Luftfahrzeugen. Der Luftaufbereiter ist dazu so angeordnet, dass sowohl die eingelassene Außenluft als auch die im Umluftkreislauf befindliche Luft aufbereitet wird. Das Luftgütesystem weist eine automatische sensorgesteuerte Umluftklappe auf, die stets geschlossen wird, wenn das Fahrzeug eine Zone stark erhöhter Luftbelastung erreicht.
Die Luftbelastung der Außenluft wird über eine sensorgesteuerte Klappeneinrichtung beeinflusst, wobei entsprechend der Schadstoff-Fracht die Ionisationsspannung gesteuert wird. Weiterhin werden die Ionen in der Fahrzeugkabine mittels eines Ionenzählers delektiert. Auf die Art der Sensoren wird nicht näher eingegangen. Der Umluftbetrieb kann durch die relativ kleinen Fahrzeugkabinen nur sehr begrenzt aufrecht erhalten werden.

Der im Patentanspruch 1 angegebenen Erfindung liegt das Problem zugrunde, wenigstens einen Raum in einem Gebäude automatisch abzuschotten und sowohl zuströmende Außenluft als auch die Luft des Raumes aufzubereiten, so dass Menschen vor auftretenden Luftverunreinigungen insbesondere schädlichen Stoffen oder Organismen geschützt werden können.

Dieses Problem wird mit den im Patentanspruch 1 aufgeführten Merkmalen gelöst.

Die Sicherheitseinrichtung für die Luft in wenigstens einem Raum eines Gebäudes, wobei der Raum über mindestens einen Anströmkanal zum Ansaugen von Außenluft und über wenigstens eine Vorrichtung zur Luftbehandlung verfügt, zeichnet sich insbesondere dadurch aus, dass Menschen in dem Raum vor Luflverunreinigungen insbesondere in Form von schädlichen Stoffen oder Organismen geschützt werden. Das wird dadurch erreicht, dass im Anströmkanal wenigstens ein Detektor für Luftverunreinigungen angeordnet ist, der über eine Steuereinrichtung mit einer bei Auftreten von Luflverunreinigungen den Anströmkanal luftdicht verschließenden Sicherheitsverschluss zusammengeschaltet ist. Vorteilhafterweise befindet sich der Detektor am Anfang des Anströmkanals während der Sicherheitsverschluss an deren Ende angeordnet ist. Am Ende des Anströmkanals befindet sich eine Vorrichtung zur Luftbehandlung. Eine Luftbehandlung der Raumluft führt dazu, dass ein Teil der Abluft des Raumes so behandelt wird, dass dieser wieder als Zuluft für den Raum verwendet werden kann. Mit derartigen Vorrichtungen zur Luftbehandlung kann der Außenluftanteil auch weniger als 10 % betragen. Erst mit einer derartigen Vorrichtung ist im Gefahrenfall eine Abkopplung von der Außenluft über einen längeren Zeitraum möglich. Ein besondere Vorteil ergibt sich dadurch, dass auch mehrere Räume beim Auftreten von Luftverunreinigungen von der Außenluft abkoppelbar sind. Mit der Vorrichtung oder den Vorrichtungen zur Luftbehandlung kann dieser Zustand auch über einen längeren Zeitraum vonstatten gehen. In diesem Zeitraum können dann weitere Maßnahmen für eine Gefahrenabwehr durchgeführt werden. Der besondere Vorteil der erfindungsgemäßen Sicherheitseinrichtung besteht darin, dass ein automatisches Abschotten des Raumes vonstatten geht und dass die Abluft des Raumes oder der Räume wieder behandelt wird.
Bei nicht vorhandenen Gefahrenquellen wird ein Anteil von Außenluft mit der Abluft des Raumes als Umluft behandelt. Dieser Anteil der benötigten Außenluft kann bis unter 10 % betragen. Im Normalbetrieb unter Zuführung von Außenluft wird ein Anteil der Abluft nicht als Umluft neu behandelt, sondern gelangt als Fortluft in die Umwelt. Dieser Fortluftkanal wird im Betrieb der Sicherheitseinrichtung verschlossen.
Die erfindungsgemäße Sicherheitseinrichtung zeichnet sich weiterhin damit dadurch aus, dass während des Betriebes weitestgehend kein Unterdruck entsteht, sondern dass über einen Zeitraum ein Druckausgleich vonstatten geht.
Die Luft des Raumes wird vorteilhafterweise durch Ionisation unter Berücksichtigung des Ozongehaltes behandelt. Die Ionisation basiert auf elektrischer Entladung in Ionisationsröhren oder durch Koronaentladungen. Die Höhe der Ionisationsleistung wird dazu abhängig von einem ersten Luftqualitätssensor, einem Luftströmungsfühler, einem Luftfeuchtefühler, einem Ozonsensor und/oder einem zweiten Luftqualitätssensor ermittelten Werten der oxidierbaren Luftbestandteilen (z.B. vaporous organic compounds - VOC), der relativen Luftfeuchte, der Strömungsgeschwindigkeit/Volumenstrom und der Belastung an Ozon der zu behandelnden Luft unter Sicherstellung einer Mindestintensität an positiven und negativen Sauerstoffionen (adäquat des Luftzustandes in der Natur) bestimmt. Die Vorrichtung zur Luftbehandlung wird dazu so geführt, dass insbesondere die Belastung der Außenluft mit flüchtigem Kohlenwasserstoff mit einem ersten Luftqualitätssensor, die Strömungsgeschwindigkeit oder der Volumenstrom der zu behandelnden Luft mit einem Luftströmungsfühler, die relative Luftfeuchte in der zu behandelnden Luft mit einem Luftfeuchtefühler, der Gehalt an Ozon in der Zuluft mit einem Ozonsensor und die oxidierbaren Luftbestandteile der Abluft und/oder der Umluft mit einem zweiten Luftqualitätssensors in dem Umluftkanal zwischen dem Raum und dem Luftaufbereitungsgerät gemessen werden und entsprechend der Werte der Messungen die Höhe der Ionisationsleistung mindestens eines oder mehrerer Ionisationsapparate so geregelt wird, dass die Mindestintensität an Sauerstoffionen bei Auftreten eines zu hohen Wertes an Ozon dieses durch die Bildung freier Radikale als auch natürlicher Sauerstoffcluster zurückgeführt wird.

Der besondere Vorteil liegt dabei darin, dass insbesondere auch der Wert des Ozons in der Zuluft entsprechend bewertet, kontrolliert und bei Erreichen/Überschreiten von Fixpunkten Signale zur Steuereinrichtung gesendet werden. Dadurch wird der Ionisationsapparat so beeinflusst, dass eine schädliche Wirkung auf im Raum sich aufhaltende Personen weitestgehend vermieden wird. Das basiert auf dem Ozonsensor in der Zuluftkanal des Raumes, der über die Steuereinrichtung mit dem Ionisationsapparat verbunden ist. Die Steuereinrichtung liefert für eine tatsächlich stabile und der Natur adäquate Zuluftionisation, wobei ein vorgegebener Ozongrenzwert nicht überschritten sowie bei einer Extremsituation Ozon eliminiert werden, optimierte Wechselimpulse, die zu dem mindestens einen Ionisationsapparat geleitet werden. Jeder Wechselimpuls ist eine volle Sinuskurve, die im Nulldurchgang angeschnitten sind. Die Frequenz wird dabei nicht verändert. Vorteilhafterweise werden mehrere Wechselimpulse (mehrere Sinuskurven) zu Paketen oder Mengen zusammengefasst. Die Paketgröße und damit die Anzahl der Wechselimpulse je Paket oder Menge stellt eine Möglichkeit dar, um die Luftionisation zu optimieren und gleichzeitig die Belastung des elektrischen Netzes zu minimieren. Die Entladungsspannung bleibt dabei konstant, so dass eine stabile Luftionisation gewährleistet wird.
Der Anteil an Ozon wird so gesenkt, dass die gewünschten und vorgegebenen Grenzwerte gewährleistet werden. In einem ersten Bereich wird dazu die Leistung des Ionisationsapparates gesenkt. Steigt der Wert des Ozongehaltes der Zuluft trotz der Absenkung der Luftionisation, so ist mindestens eine externe Ozonquelle vorhanden. In diesem Fall wird automatisch ein Modus zum Abbau des Ozones durch die Steuereinrichtung geschaltet. Werden die vorgegebenen Grenzen wieder erreicht, wird die Vorrichtung zur Luftbehandlung wieder auf Normalbetrieb geschaltet. Dabei wird das Energieniveau des Ozons so verändert, dass es zerfällt. Die Fixpunkte zur Signalisierung von bestimmten Ozonwerten werden so gewählt, dass genügend Reaktionssicherheit vorhanden ist.

Wenigstens ein weiterer Ozonsensor im Anströmkanal und/oder im Umluftkanal führt vorteilhafterweise dazu, dass sich in wenigstens einem Raum ausbildendes Ozon sofort gemessen wird. Schaltverzögerungen führen nicht zu einer Ozonzuleitung in den Raum. Das sich im Raum ausbildende Ozon stammt insbesondere von externen Quellen im Raum, wie zum Beispiel Drucker oder Kopierer. Ein weiterer Vorteil ergibt sich die Plazierung eines Ozonsensors im Anströmkanal, wobei von außerhalb eindringendes Ozon erfasst wird. Dieses Ozon stammt wiederum von externen Quellen, wie zum Beispiel Kraftfahrzeugen oder anderen Luftverschmutzungen verursachende Apparate. Der besondere Vorteil besteht darin, dass dieses Ozon abgebaut wird. Das ist bei einem verschlossenem Raum von besonderer Bedeutung. Natürlich wird auch sich anderweitig ausbildendes Ozon in der Zuluft abgebaut.
Der Ozonabbau erfolgt in den Pausen zwischen den Wechselimpulsen, der Wechselimpulsraten und/oder der Pakete von Wechselimpulsen durch den dabei stattfindenden Ausgleich der Ladungen der ionisierten Luft, wobei sich das Ozon in natürliche Sauerstoffcluster/geladene Sauerstoffmoleküle wandelt. Diese Pausen werden durch die Steuereinrichtung gezielt herbeigeführt, wobei sich der Abbau des Ozons vollzieht.

Vorteilhafte Ausgestaltungen der Erfindung sind in den Patentansprüchen 2 bis 9 angegeben.

Günstige Detektoren sind nach der Weiterbildung des Patentanspruchs 2 ein Sensor für mindestens einen chemischen Stoff, ein Sensor für einen biologischen Organismus, ein Dosimeter oder eine Vorrichtung zur Bestimmung luftgetragener Partikel. Insbesondere eine Kombination aller dieser Detektoren stellt einen optimalen Schutz von Luftverunreinigungen dar. Dabei können chemische Stoffe, biologische Organismen, energereiche Strahlung, mit energereicher Strahlung kontaminierte Partikel oder schadstoffbelastete luftgetragene Partikel erfasst werden.
Bei Vorkommen wenigstens einer dieser Stoffe, Organismen oder Partikel wird der Sicherheitsverschluss im oder am Anströmkanal betätigt, so dass der Raum luftdicht abgeschlossen wird. Natürlich können auch weitere Sensoren oder Einrichtungen zur Ermittlung von gesundheitsgefährdenden Stoffen oder Organismen als Detektor für Luftverunreinigungen eingesetzt werden.

Über ein bekanntes Lumineszenzdosimeter nach der Weiterbildung des Patentanspruchs 3 kann die auf das Lumineszenzdosimeter eingewirkte Strahlung optisch ermittelt werden. Ein besonderer Vorteil besteht darin, dass bei einem derartigen Lumineszenzdosimeter die aufgenommenen Strahlung ständig aufsummiert wird, wobei beim Auslesen die der aufgenommenen Strahlung äquivalente Lumineszenz nicht verloren geht. Die Lumineszenz wird während des Auslesens nicht gelöscht. Werden mehrere Lumineszenzdosimeter eingesetzt, kann eine kontinuierliche Kontrolle gewährleistet werden. Während des Auslesens eines ersten Lumineszenzdosimeters nimmt das zweite Lumineszenzdosimeter auch die Strahlung während des Auslesens des ersten Lumineszeuzdosimeters auf.

Der zweite Luftströmungsfühler im Anströmkanal nach der Weiterbildung des Patentanspruchs 4 wird zum Messen der Luftströmung im Anströmkanal genutzt. Daraus kann festgestellt werden ob
- der Sicherheitsverschluss in der Reaktionszeit in Form der Schaltzeiten als Verzögerungszeiten und der Trägheit des Sicherheitsverschlusses geschlossen werden konnte und/oder
- die Sicherheit in Abhängigkeit der Luftströmung gegeben ist oder Maßnahmen zum Senken der Luftströmung notwendig sind.

Eine Positionierung des Detektors für Luftverunreinigungen im Endenbereich der Ansaugöffnung des Anströmkanals nach der Weiterbildung des Patentanspruchs 5 führt vorteilhafterweise dazu, dass über die Länge des Anströmkanals die Schaltzeiten als Verzögerungszeiten und die Trägheit des Sicherheitsverschlusses bis zum luftdichten Verschluss so überbrückbar sind, dass die Luftverunreinigungen bis zum luftdichten Verschluss den Anströmkanal nicht verlassen und damit sicher nicht in den Raum gelangen können. Dabei wird nach der Weiterbildung des Patentanspruchs 6 das Volumen des Anströmkanals entsprechend einer Luftströmungsgeschwindigkeit im Anströmkanal und/oder in Abhängigkeit der Zeitverzögerungen des Detektors für Luftverunreinigungen, der Steuereinrichtung und des Antriebs einschließlich der Trägheit des Sicherheitsverschlusses ausgebildet.

Ein Federrücklaufantrieb als Antrieb für den Sicherheitsverschluss nach der Weiterbildung des Patenanspruchs 7 gewährleistet einen offenen Sicherheitsverschluss bei nicht angesteuertem Federrücklaufantrieb. Dieser Sachverhalt gewährleistet eine ökonomische Realisierung mit minimalen Energiekosten.

Über den Fortluftkanal nach der Weiterbildung des Patentanspruchs 8 gelangt ein Teil der Raumluft in die Umwelt. Die dadurch dem Raum und der Vorrichtung zur Luftbehandlung entweichende Abluft wird durch Außenluft durch den Anströmkanal ersetzt.

Mit dem wenigstens einen manuell betätigbaren Schalter, wobei der Sicherheitsverschluss nach der Betätigung geschlossen wird, nach der Weiterbildung des Patentanspruchs 9 kann nach einer Wammeldung der Sicherheitsverschluss unabhängig der Überwachung durch den Detektor für Luftverunreinigungen im Anströmkanal verschlossen werden. Dadurch wird das Gefühl der Sicherheit für die im Raum sich aufhaltenden Personen wesentlich gesteigert.

Ein Ausführungsbeispiel der Erfindung ist in den Zeichnungen dargestellt und wird im folgenden näher beschrieben.
Es zeigen:
Fig. 1 einen schematisierte Ansicht einer Sicherheitseinrichtung für die Luft in wenigstens einen Raum eines Gebäudes,
Fig. 2 eine prinzipielle Darstellung eines Paketes von zwei Wechselimpulsen zur Steuerung eines Ionisationsapparates.

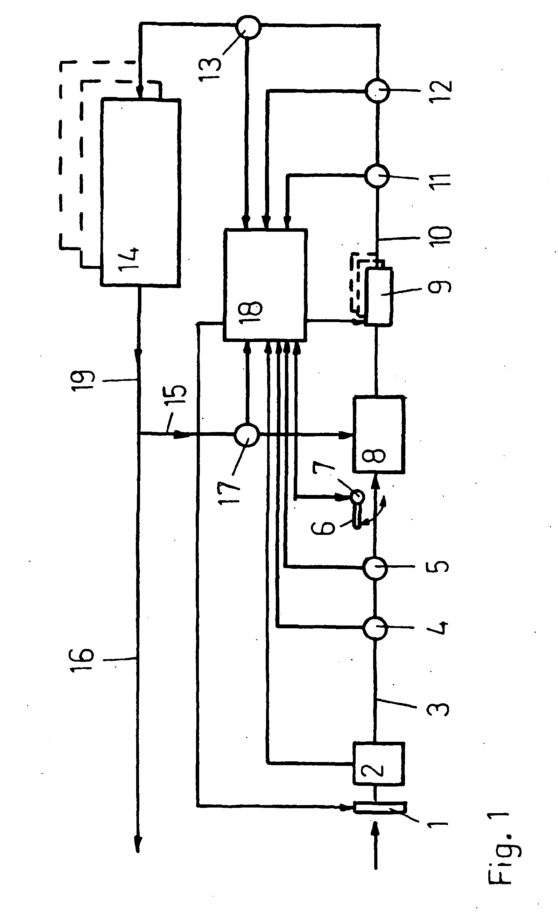

Eine Sicherheitseinrichtung für die Luft in wenigstens einem Raum 14 eines Gebäudes besteht aus einem Anströmkanal 3 zum Ansaugen von Außenluft, einer Vorrichtung zur Luftbehandlung, einer mit einem Antrieb verkoppelten Sicherheitsverschluss in Form einer Sicherheitsklappe 6, einem Abluftkanal 19 und wenigstens einem Detektor für Luftverunreinigungen 2 (Darstellung in der Fig. 1). Natürlich können auch andere Mechanismen als Sicherheitsverschlüsse eingesetzt werden, die sowohl einen offenen als auch einen geschlossenen Zustand gewährleisten.
Im Endenbereich der Öffnung des Anströmkanals 3 befindet sich der Detektor für Luftverunreinigungen 2. Dieser wandelt mindestens ein Eingangssignal um in ein Ausgangssignal, das den Wert einer oder mehrerer Zustandsgrößen wiedergibt. Das ist insbesondere ein Sensor für mindestens einen chemischen Stoff, ein Sensor für einen biologischen Organismus, ein Dosimeter oder eine Vorrichtung zur Bestimmung luftgetragener Partikel, die einzeln oder in wenigstens einer Kombination zum Einsatz kommen. Der Sensor für mindestens einen chemischen Stoff ändert seine elektrischen oder optischen Eigenschaften oder gibt ein elektrisches Signal bei Vorhandensein des chemischen Stoffes ab. Der Sensor für mindestens einen biologischen Organismus arbeitet nach dem gleichen Prinzip. Der biologische Organismus ist dabei z. B. ein Bakterium oder ein Virus. Das Dosimeter ist ein Lumineszenzdosimeter. Dabei wird unter Lumineszenz die Emission von Photonen im sichtbaren Spektralbereich in Festkörpern während oder nach einem Einwirken auf den Kristall verstanden, bei dem Energie in irgendeiner Form auf den Kristall übertragen wird. Das kann durch Einstrahlen von sichtbarem Licht oder UV-Strahlung, durch Teilchenbeschuss oder Anregung durch sonstige ionisierende Strahlung und durch chemische, mechanische oder thermische Einwirkung auf den Kristall geschehen. In der Vorrichtung zur Bestimmung luftgetragener Partikel wird insbesondere die Geometrie der Partikel erfasst. Über die Geometrie kann auf die Art der Partikel geschlossen werden, wobei die Form, die Kontur, der Flächeninhalt der Kontur, aber auch die Farbe, die Lumineszenz und/oder die Phosphoreszenz klassifiziert und entsprechend dieser Klasse die Art und deren Eigenschaften/Schädlichkeit bestimmbar wird.
Das Volumen des Anströmkanals 3 ist entsprechend einer Luftströmungsgeschwindigkeit im Anströmkanal 3 und/oder in Abhängigkeit der Zeitverzögerungen des Detektors für Luftverunreinigungen 2, der Steuereinrichtung 18 und des Antriebs 7 einschließlich der Trägheit der Sicherheitsklappe 6 ausgebildet.
Nach dem Anströmkanal 3 befindet sich die mit dem Antrieb 7 verkoppelte Sicherheitsklappe 6, die den Luftstrom bei Ansteuerung von außen luftdicht unterbricht. Der Raum 14 ist mit wenigstens einem Abluftkanal 19 versehen. Der Abluftkanal 19 ist mit der Vorrichtung zur Luftbehandlung so verbunden, dass bei offener Sicherheitsklappe 6 ein teilweiser oder bei geschlossener Sicherheitsklappe 6 ein vollständiger Umluftbetrieb vorhanden ist. Der Antrieb 7 für die Sicherheitsklappe 6 ist z. B. ein Federrücklaufantrieb. Der Antrieb 7 kann auch in einer weiteren Ausführungsform als Magnetantrieb ausgebildet sein. Weiterhin können in weiteren Ausführungsformen auch Sensoren zur Anzeige der Zustände offen oder geschlossen Bestandteile einer Sicherheitseimichtung sein. Im Anströmkanal 3 ist ein zweiter Luftströmungsfühler 4 angeordnet.
Die Vorrichtung zur Luftbehandlung ist in Strömungsrichtung der Außenluft nach der Sicherheitsklappe 6 angeordnet und besteht aus einem Luftaufbereitungsgerät 8, einem ersten Luftqualitätssensor 5 einem zweiten Luftqualitätssensor 17, einem Ionisationsapparat 9, einem ersten Luftströmungsfühler 11, einem Luftfcuchtefühler 12 und einem Ozonsensor 13. Die Luftqualitatssensoren 5, 17, die Luftströmungsfühler 4, 11, der Luftfeuchtefühler 12, der Ozonsensor 13, der Antrieb 7 für die Sicherheitsklappe 6 und der Detektor für Luftverunreinigungen 2 sind mit der Steuereinrichtung 18 zusammengeschaltet.
Die Vorrichtung zur Luftbehandlung wenigstens eines Raumes 14 erfolgt durch Luftionisation. Nach der Sicherheitsklappe 6 befindet sich das Luftaufbereitungsgerät 8, an das ein Zuluftkanal 10 und der von dem Raum 14 kommende Umlunkanal 15 angeschlossen sind. Der Zuluftstrom wird über eine Außenluftansaugung 1 in Form beispielsweise eines Zuluftventilators gefördert, der mit der Steuereinrichtung 18 zusammengeschaltet ist. Die Steuereinrichtung 18 steuert unter anderem den mindestens einen Ionisationsapparat 9 an, der in dem von dem Luftaufbereitungsgerät 8 kommenden und zu dem Raum 14 führenden Zuluftkanal 10 eingeschaltet ist.
Der Ionisationsapparat 9 besteht dabei bekannterweise aus metallischen Platten mit oder ohne Öffnungen, die in Form wenigstens eines Plattenkondensators oder mindestens eines Zylinderkondensators angeordnet sind. Die zu ionisierende Zuluft und/oder Umluft durchströmt die Platten. Während der Koronaentladung zwischen den Platten wird die Zuluft und/oder die Umluft ionisiert.
Dazu werden die Informationen in Form von elektrischen Signalen von
- dem ersten Luftqualitätssensor 5, der die dem Luftaufbereitungsgerät 8 zuströmende Außenluftqualität berücksichtigt, insbesondere die Belastung der Außenluft mit flüchtigen Kohlenwasserstoffen - vaporous organic compounds (VOC) - oder das eigentliche Oxidationspotenzial der Außenluft,
- dem zweiten Luftqualitätssensor 17, der in dem von dem Raum 14 kommende und zu dem Luftaufbereitungsgerät 8 führenden Umluftkanal 15 eingeschaltet ist und ebenfalls die flüchtigen oxidierbaren Raumluftbestandteile detektiert,
- die Luftströmungsfühler 4,11, die die Strömungsgeschwindigkeit und damit die Luftfördermenge messen,
- dem Lufrfeuchtefühler 12 und
- dem Ozonsensor 13 in der Steuereinrichtung 18 ausgewertet. Das ist insbesondere ein Mikrorechner, ein Computer oder eine programmierbare Logik, z. B. FPGA.

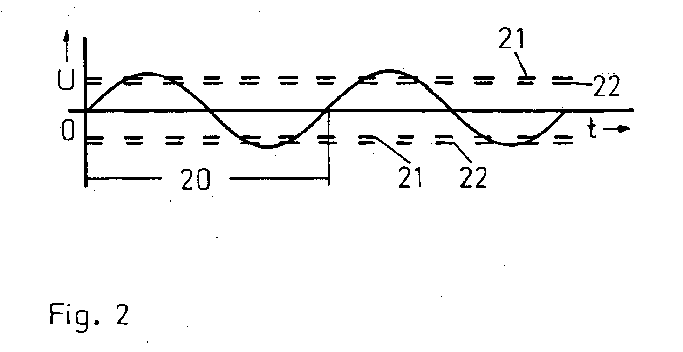

Der erste Luftströmungsfühler 11, der Luftfeuchtetühler 12 und der Ozonsensor 13 sind in den von dem Luftaufbereitungsgerät 8 kommenden und zu dem Raum 14 führenden Zuluftkanal 10 eingeschaltet.
Der erste Luftströmungsfühler 11 stellt die Strömungsgeschwindigkeit im Zuluftkanal 10 fest und der Luftfeuchtefühler 12 ermittelt die relative Luftfeuchte im Zuluftkanal 10.
Im Zuluftkanal 10 ist ferner ein Ozonsensor 13 eingeschaltet, der die Belastung an Ozon in der Zuluft feststellt und dieser Belastung äquivalente elektrische Signale der Steuereinrichtung 18 zuführt.
Die dem Ionisationsapparat 9 von der Steuereinrichtung 18 geführte elektrische Leistung wird in Abhängigkeit der Werte des ersten Luftqualitätssensors 5, des ersten Luftströmungstühlers 11, des Luftfeuchtefühlers 12, des Ozonsensors 13 und/oder des zweiten Luftqualitätssensors 17 eingestellt. Dazu werden in der Steuereinrichtung 18 die Signale von dem ersten Luftqualitätssensor 5, dem ersten Luftströmungsfühler 11, dem Luftfeuchtefühler 12, dem Ozonsensor 13 und dem zweiten Luftqualitätssensors 17 als Daten so miteinander verknüpft, dass die Steuereinrichtung 18 eine situations gerechie Leistung in Form von Wechselimpulsraten oder mehreren zu Paketen oder Mengen zusammengefassten Wechselimpulsraten an den Ioisationsapparat 9 abgibt, wenn eine höhere Luftmenge und/oder eine größere relative Luftfeuchte und/oder eine größere Raumluftbelastung mit VOC auftritt oder auftreten. In diesen Fällen erfolgt eine Vergrößerung der Wechselimpulsrate oder der Anzahl zu Paketen zusammengefasster Wechselimpulsraten. Im positiven Extremfall, z. B. bei keinen Raumluftbelastungen, wird trotzdem eine minimale Ionisationsintensität auf den Ionisationsapparat 9 geschaltet
In der Steuereinrichtung 18 erfolgt dazu
- eine Gewichtung der einzelnen Parameter und eine Verknüpfung als Summe der einzelnen Vektoren,
- eine Verknüpfung als Produkt aus den einzelnen Beträgen oder
- eine andere mathematische Behandlung,
so dass der Ionisationsapparat 9 mit einer entsprechenden optimalen oder erwünschten Leistung betrieben wird.
Der Ionisationsapparat 9 wird mit zeitlichen Folgen einer periodischen Wechselspannung gleicher oder annähernd gleicher Amplitude betrieben. Die kleinste Einheit der Folge ist dabei eine Periode der periodischen Wechselspannung als ein Wechselimpuls 20 (Darstellung in der Fig. 2). Nichtbenötigte Perioden der periodischen Wechselspannung werden abgeführt. Dadurch wird gewährleistet, dass die Spannung bei der Entladung konstant bleibt und die für den Gesamtprozess bedeutenden funktionellen Daten sowohl stabil als auch steuerbar sind. Die periodische Wechselspannung besitzt dabei eine Frequenz, die der jeweils bereitgestellten Netzfrequenz entspricht. Ein Frequenzwandler ist nicht notwendig.
Eine stabile Luftionisation und damit eine optimale Wirkungsweise, dass heißt ein hoher Anteil von positiv und negativ geladenen Sauerstoffionen mit einem hohen Bindungsbestreben - z. B. mit dem VOC-Anteil der Luft und mit einem minimalen Anteil von Radikalen in der Luft, wird nur mit einer definierten Entladungsspannung erzeugt. Diese muss weitestgehend konstant gehalten werden, so dass ein minimales Toleranzfeld eingehalten wird. Mit der Darstellung in der Fig. 2 wird nachfolgend das Verhalten der Koronaentladung beim Ändern der Entladungssannung beim Überschreiten der Grenze 21 und Unterschreiten der Grenze 22 des Toleranzfeldes zwischen den Grenzen 21, 22 einer optimalen Entladungsspannung beschrieben. Wird die Grenze 21 durch Anheben der Spannung des Ionisationsapparates 9 überschritten, wird progressiv die Ozonbelastung in der Zuluft ansteigen. Wird die Entladungsspannung dagegen unterhalb der Grenze 22 gesenkt, so ergibt sich ein Arbeitsfeld der Luftionisation, welche durch eine spontane koronare Entladung (Puffereffekt) gekennzeichnet ist, wobei ebenfalls unerwünschte Sauerstoffradikale oder Ozon freigesetzt werden. Es wird daher eine definierte Entladungsspannung im Prozess konstant gehalten. Eine situationsgerechte und stabile Luftionisation wird durch eine entsprechende Aktivierung der im Nulldurchgang angeschnittenen Sinuskurve der definierten Wechselspannung erzielt. Dabei ist eine derartige Sinuskurve ein jeweiliger Wechselimpuls 20, der den Ionisationsapparat 9 in Funktion setzt. Zur weiteren Optimierung der Wirkungsweise der Luftionisation ist die Steuereinrichtung 18 so ausgelegt, dass zusätzlich die Wechselimpulsraten zu sinnvollen Paketen oder Mengen bestimmter Anzahl von Wechselimpulsen zusammengefasst werden können.
Die Signale des Ozonsensors 13 werden wie folgt ausgewertet oder im Prozess genutzt:
- von 0 bis 0,06 ppm Ozon-Anteil im Zuluftstrom keine Einflussnahme,
- größer/gleich 0,06 ppm Ozon-Anteil Absenkung der momentanen Ionisationsleistung auf 50 %,
- bei weiterem Anstieg des Ozon-Anteils liegt eine externe Ozonquelle vor und es wird die Maßnahme zum Abbau des Ozons eingeleitet.
Diese besteht darin, dass gleichzeitig der Abstand der Wechselimpulse, der Wechselimpulsraten und/oder der Pakete von Wechselimpulsen verändert wird, wobei ein dabei in den Pausen stattfindender Ausgleich der Ladungen der ionisierten Luft zu einer Wandlung des Ozons in natürliche Sauerstoffcluster/ geladene Sauerstoffmoleküle führt. Der Energieausgleich entspricht weitestgehend dem der Natur. Natürlich existieren Sauerstoff, geladene Sauerstoffmoleküle und Ozon in der Atmosphäre. Ozon ist eine labile Verbindung, das bei Energieaufnahme in geladene bipolare Sauerstoffmoleküle zerfällt. Dieser Prozess wird vorteilhafterweise in den Pausen bewusst zum Ozonabbau genutzt.
Der Betrieb der Vorrichtung zur Luftbehandlung erfolgt weiterhin so, dass eine Mindest-Ionisationsleistung aufrechterhalten wird, auch wenn extrem niedrige Prozessdaten vorliegen. Das ist insbesondere dann der Fall, wenn der erste Luftqualitätssensor 5, der erste Luftströmungsfühler 11, der Luftfeuchtefühler 12, der Ozonsensor 13 und der zweite Luftqualitätssensor 17 der Steuereinrichtung 18 signalisieren, dass an sich keine Ionisation erfolgen müsste. Dabei wird dem adäquaten Natureffekt entsprochen.
Während des Betriebs der Vorrichtung zur Luftbehandlung wird über den Fortluftkanal 16 nur eine geringe Menge an Fortluft abgeführt, der eine entsprechende Menge an Außenluft gegenübersteht, die dann über den Anströmkanal 3 zugeführt wird.
Die Steuereinrichtung 18 und/oder der Antrieb sind mit mindestens einem manuell betätigbaren Schalter so versehen, dass der Sicherheitsverschluss nach der Betätigung geschlossen wird.

In weiteren Ausführungsformen kann die Steuereinrichtung 18 auch mit weiteren die Zusammensetzung der Raumluft überwachenden Sensoren oder Einrichtungen zusammengeschaltet sein. Um die Zeitdauer des luftdichten Verschlusses zu erhöhen, können auch mehrere Räume über Kanäle verbunden sein, so dass auch ein Luftausgleich zwischen den Räumen durchführbar ist.

Im Anströmkanal 3 und/oder im Umluftkanal 15 ist wenigstens ein weiterer Ozonsensor angeordnet. Dieser wenigstens eine weitere Ozonsensor ist mit der Steuereinrichtung 18 so verbunden ist, dass dieser entsprechend der Höhe des Gehalts an Ozon ein elektrisches Signal oder ein entsprechend eines vorbestimmbaren Wertes oder mehrerer vorbestimmbarer Werte des Gehalts an Ozon ein elektrisches Signal liefert.

In einer weiteren Ausführungsform einer Sicherheitseinrichtung für die Luft in wenigstens einem Raum eines Gebäudes können die Vorrichtung zur Luftbehandlung mit einer Steuereinrichtung und der wenigstens eine Detektor für Luftverunreinigungen und der Antrieb für den Sicherheitsverschluss mit einer weiteren Steuereinrichtung zusammengeschaltet sein.
In weiteren Ausführungsformen können natürlich auch andere Vorrichtungen zur Luftbehandlung ein Bestandteil der erfindungsgemäßen Sicherheitseinrichtung sein.

## Patentansprüche

1. Sicherheitsvorrichtung für die Luft in wenigstens einen Raum (14) eines Gebäudes mit
- mindestens einem Anströmkanal (3) zum Ansaugen von Außenluft, wobei im Anströmkanal (3) wenigstens ein mindestens ein Eingangssignal in ein den Wert einer oder mehrerer Zustandsgrößen wiedergebendes Ausgangssignal wandelnder Detektor für Luftverunreinigungen (2) angeordnet ist,
- einem mit einem Antrieb verkoppelter Sicherheitsverschluss, so dass dieser Sicherheitsverschluss den Anströmkanal (3) oder den Eingang der Vorrichtung zur Luftbehandlung entweder wenigstens teilweise nicht oder luftdicht verschließt,
- einer Vorrichtung zur Luftbehandlung in Strömungsrichtung der Außenluft nach dem Sicherheitsverschluss bestehend aus einem Luflaufbereitungsgerät (8), einem ersten Luftqualitätssensor (5), einem zweiten Luftqualitätssensor (17), einem Ionisationsapparat (9), einem ersten Luftströmungsfühler (11), einem Luftfeuchtefühler (12) und einem Ozonsensor (13), wobei im Anströmkanal (3) und/oder im Zuluftkanal (10) zu dem Luftaufbereitungsgerät (8) der erster Luftqualitätssensor (5) geschalten ist, sich im Raum (14) und/oder in dem Abluftkanal (19) des Raumes (14) und/oder in einem Umluftkanal (15) zwischen dem Abluftkanal (19) und dem Luftaufbereitungsgerät (8) der zweite Luftqualitätssensor (17) befindet, der Zuluftkanal (10) zwischen dem Luftaufbereitungsgerät (8) und dem Raum (14) mit mindestens einem Ionisationsapparat (9), dem ersten Luftströmungsfühler (11), dem Luftfeuchtefühler (12) und dem Ozonsensor (13) versehen sind,
- wenigstens einem weiteren Ozonsensor im Anströmkanal (3) und/oder im Umluftkanal (15),
- wenigstens einen Abluftkanal (19) für den Raum (14), wobei der Abluftkanal (19) mit der Vorrichtung zur Luftbehandlung so verbunden ist, dass bei offenem Sicherheitsverschluss ein teilweiser oder bei geschlossenem Sicherheitsverschluss ein vollständiger Umluftbetrieb vorhanden ist und dass der Detektor für Luftvcrunreinigungen (2), die Vorrichtung zur Luftbehandlung (5, 8, 9, 11, 12, 13, 17), der wenigstens eine weitere Ozonsensor und der Antrieb mit wenigstens einer Steuereinrichtung (18) zusammengeschaltet sind, wobei diese Steuereinrichtung eine in Abhängigkeit der Werte des ersten Luftqualitätssensor (5), des ersten Luftströmungsfühlers (11), des Luftfeuchtefühlers (12) und/oder des zweiten Luftqualiätssensors (17) automatisch dem lonisationapparat (9) elektrische Leistung zuführende, diese Steuereinrichtung eine bei Lieferung eines elektrischen Signals durch Auftreten von Ozon eines bestimmten Wertes durch die Ozonsensoren die momentane lonisationsleistung reduzierende und diese Steuereinrichtung eine bei Lieferung eines elektrischan Signals entsprechend entweder einer weiteren Steigerung oder gleich bleibendem bestimmten Wert des Ozongebaltes durch die Ozonsensoren den Abstand der Wechselimpulse, der Wechselimpulsraten und/oder der Pakete von Wechselimpulsen einer zeitlich zugeschalteten periodischen Wechselspannung verändemde Steuereunrichtung (18) ist.

2. Sicherheitseinrichtung nach Patentanspruch 1, **dadurch gekennzeichnet, dass** der Detektor für Luftverunreinigungen (2) ein Sensor für mindestens einen chemischen Stoff, ein Sensor für wenigstens einen biologischen Organismus, ein Dosimeter oder eine Vorrichtung zur Bestimmung luftgetragener Partikel ist.

3. Sicherheitseinrichtung nach Patentanspruch 2, **dadurch gekennzeichnet, dass** das Dosimeter ain Lumineszenzdosimeter ist.

4. Sicherbeitseinrichtung nach Patentanspruch 1, **dadurch gekennzeichnet, dass** im Anströmkanal (3) ein zweiter Luftströmungsfühler (4) angeordnet ist und dass der zweite Luftströmungsfühler (4) mit der Steuereinrichtung (18) zusammengeschaltet ist.

5. Sicherheitseinrichtung nach den Patentansprüchen 1 bis 4, **dadurch gekennzeichnet, dass** sich der Detektor für Luftverunreinigungen (2) im Endenbereich der Ansaugöffnung des Anströmkanals (3) und/oder in der Außenluftansaugung (1) befindet.

6. Sicherheitseinrichtung nach den Patentansprüchen 1 bis 5, **dadurch gekennzeichnet, dass** das Volumen des Anströmkanals (3) entsprechend einer Luftströmungsgeschwindigkeit im Anströmkanal (3) und/oder in Abhängigkeit der Zeitverzögerungen des Detektors für Luftverunreinigungen (2), der Steuereinrichtung (18) und des Antriebs einschließlich der Trägheit des Sicherheitsverschlusses ausgebildet ist.

7. Sicherheitseinrichtung nach Patentanspruch 1, **dadurch gekennzeichnet, dass** der Antrieb ein Federrücklaufantrieb ist

8. Sicherheitseinrichtung nach Patentanspruch 1, **dadurch gekennzeichnet, dass** der Raum (14) mit einem Fortluftkanal (16) direkt oder indirekt über einen Umluftkanal (15) verfügt, dass der Fortluftkanal (16) über eine angetriebene Klappe oder ein Ventil verschließbar ist und dass der Antrieb der Klappe oder die Betätigungseinrichtung des Ventils mit der Steuereinrichtung (18) zusammengeschaltet ist.

9. Sicherheitseinrichtung nach Patentanspruch 1, **dadurch gekennzeichnet, dass** die Steuereinrichtung (18) und/oder der Antrieb mit mindestens einem manuell betätigbaren Schalter so versehen ist, dass der Sicherheitsverschluss nach der Betätigung geschlossen wird.

## Claims

1. Safety device for the air in at least one room (14) of a building including
- at least one intake channel (3) for drawing in outside air, whereby at least one detector for air contaminants (2) which converts at least one input signal into an output signal that represents one or several state variables is arranged in the intake channel (3),
- a safety closure coupled to a drive so that said safety closure either at least partly does not close or airtightly closes the intake channel (3) or the inlet of the device for air treatment,
- a device for air treatment in direction of flow of the outside air downstream of the safety closure, comprising an air preparation device (8), a first air quality sensor (5), a second air quality sensor (17), an ionization apparatus (9), a first air flow sensor (11), an air humidity sensor (12) and an ozone sensor (13), the first air quality sensor (5) being arranged in the intake channel (3) and/or in the supply air duct (10) to the air preparation device (8), the second air quality sensor (17) being arranged in the room (14) and/or in the exhaust air duct (19) of the room (14) and/or in the recirculating air duct (15) between the exhaust air duct (19) and the air preparation device (8), the supply air duct (10) between the air preparation device (8) and the room (14) being provided with at least one ionization apparatus (9), the first air flow sensor (11), the air humidity sensor (12) and the ozone sensor (13),
- at least one further ozone sensor in the intake channel (3) and/or the recirculating air duct (15),
- at least one exhaust air duct (19) for the room (14), the exhaust air duct (19) being connected to the device for air treatment such that with the safety closure in the open position, a partial recirculating operation occurs or with the safety closure in the closed position, a complete recirculating operation is present, and that the detector for air contaminants (2), the device for air treatment (5, 8, 9, 11, 12, 13, 17), the at least one further ozone sensor and the drive are connected to at least one control device (18), said control device (18) automatically supplying electric power to the ionization apparatus (9) dependent on the values of the first air quality sensor (5), the first air flow sensor (11), the air humidity sensor (12) and/or the second air quality sensor (17), said control device (18) reducing the instantaneous ionizisation output when the ozone sensors supply an electrical signal due to the presence of a certain value of ozone, said control device (18) changing the spacing of the alternating pulses, the alternating pulse rates and/or the alternating pulse bursts of a periodic alternating voltage timedly switched on when the ozone sensors supply an electrical signal according to either a further increase or constant certain value of the ozone content.

2. Safety device to claim 1 **characterized by that** the detector for air contaminants (2) is a sensor for at least one chemical substance, a sensor for at least one biological organism, a dosimeter or a device for the determination of airborne particles.

3. Safety device to claim 2 **characterized by that** the dosimeter is a luminiscence dosimeter.

4. Safety device to claim 1 **characterized by that** a second air flow sensor (4) is arranged in the intake channel (3), the second air flow sensor (4) being connected to the control device (18).

5. Safety device to claims 1 to 4 **characterized by that** the detector for air contaminants (2) is positioned in the end region of the draw in opening of the intake channel (3) and/or in the outside air draw in (1).

6. Safety device to claims 1 to 5 **characterized by that** the volume of the intake channel (3) is established according to an air flow speed in the intake channel (3) and/or dependent on the time delays of the detector for air contaminants (2), the control device (18) and the drive, inclusive of the response time of the safety closure.

7. Safety device to claim 1 **characterized by that** the drive is a spring return drive.

8. Safety device to claim 1 **characterized by that** the room (14) with an exit air duct (16) is directly or indirectly provided with a recirculating air duct (15), the exit air duct (16) being closable by using a powered flap or a valve, the drive of the flap or the actuator of the valve switched to the control device (18).

9. Safety device to claim 1 **characterized by that** the control device (18) and/or the drive is provided with at least one manually actuatable switch such that the safety closure is closed after the actuation thereof.

## Revendications

1. Dispositif de sécurité destiné à l'air d'au moins un local (14) d'un bâtiment, comprenant
- au moins un canal d'entrée d'air (3) pour aspirer de l'air extérieur, au moins un détecteur pour des impuretés de l'air (2) convertissant au moins un signal d'entrée en un signal de sortie représentant la valeur d'une ou plusieurs variables d'état étant disposé dans le canal d'entrée d'air (3),
- une fermeture de sécurité couplée à un entraînement de façon que cette fermeture de sécurité ne ferme pas au moins partiellement ou ferme de manière étanche à l'air le canal d'entrée d'air (3) ou l'entrée du dispositif pour le traitement d'air,
- un dispositif pour le traitement d'air en aval de la fermeture de sécurité dans le sens d'écoulement de l'air extérieur, composé d'un appareil de traitement d'air (8), d'un premier capteur de qualité d'air (5), d'un deuxième capteur de qualité d'air (17), d'un appareil d'ionisation (9), d'un premier capteur d'écoulement d'air (11), d'un capteur d'humidité d'air (12) et d'un capteur d'ozone (13), le premier capteur de qualité d'air (5) étant monté dans le canal d'entrée d'air (3) et/ou dans le canal d'amenée d'air (10) vers l'appareil de traitement d'air (8), le deuxième capteur de qualité d'air (17) se trouvant dans le local (14) et/ou dans le canal de sortie d'air (19) du local (14) et/ou dans un canal de recirculation d'air (15) entre le canal de sortie d'air (19) et l'appareil de traitement d'air (8), le canal d'amenée d'air (10) entre l'appareil de traitement d'air (8) et le local (14) étant pourvu d'au moins un appareil d'ionisation (9), du premier capteur d'écoulement d'air (11), du capteur d'humidité d'air (12) et du capteur d'ozone (13),
- au moins un autre capteur d'ozone dans le canal d'entrée d'air (3) et/ou dans le canal de recirculation d'air (15),
- au moins un canal de sortie d'air (19) pour le local (14), le canal de sortie d'air (19) étant relié au dispositif de traitement d'air de façon qu'en cas de fermeture de sécurité ouverte, une recirculation d'air partielle ou en cas de fermeture de sécurité fermée une recirculation d'air complète soit présente et que le détecteur pour des impuretés de l'air (2), le dispositif de traitement d'air (5, 8, 9, 11, 12, 13, 17), ledit au moins un autre capteur d'ozone et l'entraînement soient interconnectés avec au moins un dispositif de commande (18), ce dispositif de commande amenant automatiquement une puissance électrique à l'appareil d'ionisation (9) en fonction des valeurs du premier capteur de qualité d'air (5), du premier capteur d'écoulement d'air (11), du capteur d'humidité d'air (12) et/ou du deuxième capteur de qualité d'air (17), ce dispositif de commande réduisant la puissance d'ionisation momentanée en cas de délivrance d'un signal électrique dû à la présence d'ozone d'une certaine valeur par les capteurs d'ozone et ce dispositif de commande modifiant l'espacement des impulsions alternatives, des taux d'impulsions alternatives et/ou des paquets d'impulsions alternatives d'une tension alternative périodique activée temporellement en cas de délivrance d'un signal électrique correspondant soit à une augmentation supplémentaire, soit à une certaine valeur inchangée de la teneur en ozone par les capteurs d'ozone.

2. Dispositif de sécurité selon la revendication 1, **caractérisé en ce que** le détecteur pour des impuretés de l'air (2) est un capteur pour au moins une substance chimique, un capteur pour au moins un organisme biologique, un dosimètre ou un dispositif de détermination de particules aériennes.

3. Dispositif de sécurité selon la revendication 1, **caractérisé en ce que** le dosimètre est un dosimètre à luminescence.

4. Dispositif de sécurité selon la revendication 1, **caractérisé en ce qu'**un deuxième capteur d'écoulement d'air (4) est disposé dans le canal d'entrée d'air (3) et que le deuxième capteur d'écoulement d'air (4) est interconnecté avec le dispositif de commande (18).

5. Dispositif de sécurité selon les revendications 1 à 4, **caractérisé en ce que** le détecteur pour des impuretés de l'air (2) se trouve dans la zone d'extrémité de l'ouverture d'aspiration du canal d'entrée d'air (3) et/ou dans l'aspiration d'air extérieur (1).

6. Dispositif de sécurité selon les revendications 1 à 5, **caractérisé en ce que** le volume du canal d'entrée d'air (3) est conçu en fonction d'une vitesse d'écoulement d'air dans le canal d'entrée d'air (3) et/ou en fonction des retards de temps du détecteur pour des impuretés de l'air (2), du dispositif de commande (18) et de l'entraînement, y compris l'inertie de la fermeture de sécurité.

7. Dispositif de sécurité selon la revendication 1, **caractérisé en ce que** l'entraînement est un entraînement à rappel par ressort.

8. Dispositif de sécurité selon la revendication 1, **caractérisé en ce que** le local (14) avec un canal d'évacuation d'air (16) dispose directement ou indirectement d'un canal de recirculation d'air (15), que le canal d'évacuation d'air (16) peut être fermé par un volet ou un clapet entraîné et que l'entraînement du volet ou le dispositif de commande du clapet est interconnecté avec le dispositif de commande (18).

9. Dispositif de sécurité selon la revendication 1, **caractérisé en ce que** le dispositif de commande (18) et/ou l'entraînement sont pourvus d'au moins un commutateur actionnable manuellement de façon que la fermeture de sécurité soit fermée après l'actionnement.
